# DEMANDE DE BREVET EUROPEEN

(11) **EP 0 545 772 A1**
(43) Date de publication de la demande: **09.06.1993**
(21) Numéro de dépôt: 92403151.1
(22) Date de dépôt: 24.11.1992
(51) Int. Cl.: A61K 37/36

(54) **Composition pharmaceutique contenant de l'EGF pour le traitement des pathologies bronchopulmonaires**

(30) Priorité: 02.12.1991 FR 9114886
(71) Demandeur: SYNTHELABO, F-92350 Le Plessis Robinson (FR); INSTITUT NATIONAL DE LA SANTE ET DE LA RECHERCHE MEDICALE (INSERM), 75654 Paris Cédex 13 (FR)
(72) Inventeur: Zahm, Jean-Marie, INSERM U 314, F-51092 Reims Cédex (FR); Puchelle, Edith, INSERM U 314, F-51092 Reims Cédex (FR); Pierrot, Denis, INSERM U 314, F-51092 Reims Cédex (FR); Benali, Rachid, INSERM U 314, F-51092 Reims Cédex (FR); Moreau,Aline, F-75007 Paris (FR)
(74) Mandataire: Thouret-Lemaitre, Elisabeth

(57) **Abrégé**

Composition pharmaceutique contenant de l'EGF (Epidermal Growth Factor) utile pour le traitement des pathologies bronchopulmonaires s'accompagnant de lésions de l'épithélium bronchique.

## Description

La présente invention a pour objet une composition pharmaceutique contenant de l'EGF utile pour le traitement des pathologies bronchopulmonaires s'accompagnant de lésions de l'épithélium bronchique (intoxications accidentelles, infections bronchopulmonaires, bronchites chroniques, emphysemes, syndrome de détresse respiratoire).

Le facteur de croissance EGF (Epidermal Growth Factor) est un polypeptide synthétisé dans de nombreux organes et dont l'action stimulante sur la prolifération cellulaire a été démontrée sur des cellules gliales, des cellules mésenchymateuses et des cellules épithéliales en culture. Au niveau de l'épithélium respiratoire, St. George et al (Am. J. Respir. Cell Mol. Biol., 1991, 4, 95-101) ont montré que l'EGF est impliqué dans le développement foetal du système sécrétoire trachéo-bronchique du singe. Stahlman et al. (Lab. Invest., 1989, 60, 539-547) ont mis en évidence par immunocytochimie, la présence d'EGF dans le tissu respiratoire au cours du développement foetal chez l'homme. Par contre chez l'animal, il a été montré que l'EGF n'a pas d'effet sur la prolifération de cellules épithéliales de trachée de chien en culture mais qu'il favorise la réponse de ces mêmes cellules à une stimulation par des béta-adrénergiques (Van Scott, Am. J. Physiol., 1988, 255, C237-C245). A l'opposé, Wu et al. (Am. Rev. Respir. Dis., 1985, 132, 311-320) ont rapporté que l'omission d'EGF dans le milieu de culture induit une diminution de la prolifération de cellules épithéliales nasales humaines. La Demanderesse a étudié l'effet de l'EGF sur la croissance, la différentiation et l'activité fonctionnelle de cellules respiratoires humaines en culture. De plus, à partir de modèles in vitro de lésions de l'épithélium respiratoire, la Demanderesse a mis en évidence un effet potentiel de l'EGF sur la réparation de ces lésions.

Des cultures de cellules épithéliales respiratoires sont obtenues à partir de polypes nasaux, selon la technique des explants (croissance des cellules autour de l'explant) et selon la technique de cellules dissociées mises en culture dans une matrice de collagène tri-dimensionnelle (lattis de collagène).
Les explants sont mis en culture en présence de différentes concentrations (0, 5, 10 et 20 ng/ml) d'EGF (EGF de glandes sous-maxiliaires de souris, réf. E 4127, Sigma). La surface de la croissance autour des explants est quantifiée après 3 et 4 jours de culture. Le pourcentage de la surface de la croissance couverte par des cellules ciliées, ainsi que la fréquence de battement ciliaire ont été mesurés après 3 jours de culture. Dans la technique de culture en lattis de collagène, le nombre de cellules en culture a été quantifié jusqu'à 11 jours après la mise en culture, et ceci en absence et en présence d'EGF (10 ng/ml).
Les résultats obtenus montrent que l'augmentation de concentration en EGF dans le milieu de culture (jusqu'à 20 ng/ml) se traduit par une augmentation significative de la surface de la croissance (p < 0,05) autour des explants (la vitesse de prolifération cellulaire est plus importante lorsque les cellules cultivées dans un lattis de collagène sont en contact avec de l'EGF à une concentration de 10 ng/ml, comparativement à des cultures sans EGF. Après 8 jours de culture, le nombre de cellules est 2 fois plus important en présence d'EGF. La présence d'EGF dans le milieu de culture, lorsque les cellules épithéliales respiratoires sont cultivées dans un lattis de collagène, favorise la formation de structures tri-dimensionnelles de type tubulaire. Ce processus de développement de tubules a été décrit au cours de la morphogénèse glandulaire bronchique. En absence d'EGF, les cellules épithéliales ont tendance à former un tapis cellulaire bi-dimensionnel.

En ce qui concerne l'activité fonctionnelle des cellules ciliées, on note un pourcentage plus important (43 %) de surface de croissance couverte par des cellules ciliées en présence d'EGF à 20ng/ml, comparé au pourcentage obtenu lors de la culture en absence d'EGF (30 %). La fréquence de battement ciliaire est également significativement plus élevée (14,5 Hz) en présence d'EGF à 20 ng/ml, comparativement à la fréquence ciliaire mesurée en absence d'EGF (12,6 Hz).

L'étude de la réparation de lésions de l'épithélium respiratoire a été effectuée sur un modèle in vitro. Trente à 50 cellules sont détachées de la culture à l'aide d'un micromanipulateur, de façon à mettre à nu la matrice de collagène sur laquelle les cellules épithéliales prolifèrent. L'évolution de cette zone lésée est suivie en fonction du temps sur des cultures en présence d'EGF (10 ng/ml) ou en absence d'EGF. Dans la technique de culture de cellules dissociées dans un lattis de collagène, l'effet de l'EGF sur la rétraction du lattis de collagène a été étudié, après 8 jours de culture. Cette rétraction est quantifiée par la mesure de la surface du lattis de collagène.
En présence d'une concentration d'EGF de 10 ng/ml, les lésions réparent plus rapidement qu'en absence d'EGF (avec EGF, pente de la droite de régression = -71,8 ; sans EGF, pente de la droite de régression =-42,3).
En ce qui concerne la rétraction des lattis de collagène par les cellules épithéliales respiratoires de surface on constate qu'en présence d'EGF, après 8 jours de culture, la surface du lattis est significativement plus faible (660 mm²) que la surface obtenue sans EGF (800 mm²). Il est admis que ce processus de rétraction d'un lattis de collagène par des cellules en culture est représentif des phénomènes de cicatrisation de lésions in vivo (Ehrlich, Tissue and Cell, 1990). Cette rétraction par des cellules respiratoires n'a jamais été montrée jusqu'à présent.

Les Demanderesses ont également étudié,l'effet-dose de l'EGF sur la réparation de lésions, avec une quantification de l'importance de la stimulation comparativement à un témoin, ainsi que des données comparatives entre l'EGF obtenu à partir de glandes submaxillaires de souris et l'EGF recombinant humain (Sigma, Réf E-3264).

### Modèle in vitro de lésion de l'épithélium respiratoire :

Des cellules épithéliales de surface dissociées à partir de polype nasal sont mises en culture sur une matrice de collagène dans du milieu de culture RPMI supplémenté par l'hydrocortisone, l'acide rétinoïque, l'insuline et l'EGF (epidermal growth factor). Après environ 3 jours de culture, lorsque les cellules sont confluentes, le milieu de culture supplémenté est remplacé par du milieu RPMI non supplémenté. Après 12 heures d'incubation en présence de milieu non supplémenté, la culture est placée sur la platine d'un microscope inversé équipé d'un micromanipulateur. Un microcapillaire, guidé par le micromanipulateur, permet de détacher des cellules, créant ainsi une lésion ayant une surface d'environ 100000 µm². Le milieu de culture non supplémenté est alors remplacé par du milieu de culture ne contenant que la molécule dont on veut étudier l'effet sur la réparation. Des enregistrements vidéo de la culture lésée sont ensuite effectués toutes les heures. A partir de ces enregistrements vidéo, un analyseur d'images permet de suivre l'évolution de la surface de la lésion en fonction du temps. Une régression linéaire entre la surface de la lésion (exprimée en µm²) et le temps (exprimé en heures) permet de calculer la pente de la droite de régression obtenue, pente correspondant à la diminution de la surface de la lésion par unité de temps. Ce dernier paramètre correspond à l'indice de réparation (plus l'indice est élevé, plus la réparation est rapide).

### Effet-dose de l'EGF :

Huit polypes nasaux ont été utilisés pour mettre en culture des cellules épithéliales respiratoires (5 boîtes de culture par polype). Dans chacune de ces boîtes, les cultures lésées sont incubées soit en présence de milieu de culture non supplémenté, soit en présence de milieu de culture supplémenté en EGF à différentes concentrations (5, 10, 20 ou 50 ng/ml). A partir de l'évolution de la surface en fonction du temps, nous avons calculé l'indice de réparation. Cet indice est ensuite exprimé par rapport à l'indice de réparation obtenu avec du milieu de culture sans EGF utilisé comme témoin. Les résultats obtenus en fonction de la concentration en EGF sont résumés sur la figure 1. On observe une augmentation systématique de l'indice de réparation des lésions en présence d'EGF, et ce quelle que soit la concentration. A une concentration de 5 et 10 ng/ml, l'EGF stimule la réparation de 38 % et 36 %, respectivement. A la concentration de 20 ng/ml, la stimulation est de 49 %, alors qu'à 50 ng/ml elle n'est plus que de 25 %.
En conclusion, l'EGF stimule in vitro la réparation de lésion de l'épithélium respiratoire. Cette stimulation est maximale à la concentration de 20 ng/ml et elle est moins importante pour des concentrations supérieures à 20 ng/ml.

### Comparaison de l'EGF de glande submaxillaire de souris et de l'EGF recombinant humain:

Cette étude a été réalisée dans les mêmes conditions expérimentales que ci-dessus. L'indice de réparation des lésions a été quantifié en présence de milieu de culture non supplémenté, et en présence de milieu de culture supplémenté soit avec de l'EGF de glande submaxillaire de souris (m-EGF : 10 ng/ml), soit d'EGF recombinant humain (h-EGF : 10 ng/ml). Il n'y a pas de différence significative entre l'indice de réparation obtenu avec m-EGF (11215 µm²/h) et l'index de réparation obtenu avec H-EGF (11492 µm²/h), tous deux étant significativement plus élevés que l'index de réparation obtenu sans EGF (6235 µm²).
En conclusion, l'EGF obtenu à partir de glandes submaxillaires de souris et l'EGF recombinant humain ont le même effet sur la réparation de lésions de l'épithélium respiratoire.
L'EGF favorise la réparation de lésions de l'épithélium respiratoire selon un effet qui est dose-dépendant. L'effet maximal est obtenu à une concentration de 20 ng/ml d'EGF, avec une stimulation de près de 50 % de l'indice de réparation comparativement à un témoin sans EGF. L'étude comparative entre l'EGF de glandes submaxillaires de souris et l'EGF recombinant humain ne montre pas de différence significative entre l'effet de ces deux formes d'EGF.
. L'EGF stimule la croissance et la différentiation des cellules épithéliales respiratoires humaines et ceci suivant un effet dose-dépendant : augmentation de la prolifération cellulaire et de l'activité fonctionnelle des cellules ciliées
. L'EGF favorise la cicatrisation de lésions de l'épithélium respiratoire.
. L'EGF favorise le développement de tubules observé au cours de la morphogénèse glandulaire bronchique.
. L'EGF stimule l'activité ciliaire in vitro.

L'EGF peut donc favoriser la réparation de l'épithélium bronchique après toute agression de celui-ci par un toxique, une infection, un oxydant etc.

Les compositions pharmaceutiques de l'invention qui contiennent de l'EGF peuvent être administrées par voie intraveineuse ou par inhalation et se présenter sous la forme de solutions, solutions pour nébulisations, sprays, etc...

## Revendications

1. Composition pharmaceutique contenant de l'EGF (Epidermal Growth Factor) utile pour le traitement des pathologies bronchopulmonaires s'accompagnant de lésions de l'épithélium bronchique.

2. Utilisation de l'EGF (Epidermal Growth Factor) pour la fabrication de moyens destinés à traiter les pathologies bronchopulmonaires s'accompagnant de lésions de l'épithélium bronchique.
